(19) Europäisches Patentamt
European Patent Office
Office européen des brevets

(11) Veröffentlichungsnummer: **0 462 156 B1**

(12) # EUROPÄISCHE PATENTSCHRIFT

(45) Veröffentlichungstag der Patentschrift: **10.08.94**

(51) Int. Cl.[5]: **C07D 405/12**, C07D 317/30, C07D 317/72, C09K 19/06, C09K 19/34, G02F 1/13

(21) Anmeldenummer: **90904314.3**

(22) Anmeldetag: **07.03.90**

(86) Internationale Anmeldenummer: **PCT/EP90/00369**

(87) Internationale Veröffentlichungsnummer: **WO 90/10629 (20.09.90 90/22)**

Die Akte enthält technische Angaben, die nach dem Eingang der Anmeldung eingereicht wurden und die nicht in dieser Patentschrift enthalten sind.

(54) **OPTISCH AKTIVE, EINEN MESOGENEN REST TRAGENDE DIOXOLANYLACRYL- UND DIOXOLANYLPROPIONSÄUREESTER, EIN VERFAHREN ZU IHRER HERSTELLUNG UND IHRE VERWENDUNG ALS DOTIERSTOFFE IN FLÜSSIGKRISTALLMISCHUNGEN.**

(30) Priorität: **09.03.89 DE 3907601**

(43) Veröffentlichungstag der Anmeldung: **27.12.91 Patentblatt 91/52**

(45) Bekanntmachung des Hinweises auf die Patenterteilung: **10.08.94 Patentblatt 94/32**

(84) Benannte Vertragsstaaten: **AT BE CH DE FR GB IT LI NL SE**

(56) Entgegenhaltungen:
**EP-A- 0 234 437**
**EP-A- 0 351 746**
**DE-A- 3 713 273**

(73) Patentinhaber: **HOECHST AKTIENGESELLSCHAFT**
**D-65926 Frankfurt (DE)**

(72) Erfinder: **MÜLLER, Ingrid**
**Am Pfingstbrunnen 1**
**D-6238 Hofheim am Taunus (DE)**
Erfinder: **DÜBAL, Hans-Rolf**
**Heuhohlweg 6**
**D-6240 Königstein/Taunus (DE)**
Erfinder: **ESCHER, Klaus**
**Amselweg 3**
**D-6109 Mühltal (DE)**
Erfinder: **HEMMERLING, Wolfgang**
**Billtalstrasse 32**
**D-6231 Sulzbach (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

**Chemical Abstracts, Band 109, 1988, Zusammenfassung-Nr. 139700r, Columbus, Ohio, US; I.V. Vladimirskii et al.: "Induction of the helical structure in nematic liquid crystals by chiral dopants"**

Erfinder: **ILLIAN, Gerhard**
**Farbenstrasse 62**
**D-6230 Frankfurt am Main 80 (DE)**
Erfinder: **MURAKAMI, Mikio**
**New Town Bldg. 202**
**8-1, Minami 2-chome**
**Kakegawa-shi Shizuoka-ken (JP)**
Erfinder: **OHLENDORF, Dieter**
**Am Kühlen Grund 4**
**D-6237 Liederbach (DE)**
Erfinder: **WINGEN, Rainer**
**Brunnenstrasse 1**
**D-6234 Hattersheim am Main (DE)**

## Beschreibung

Flüssigkristalle haben insbesondere im letzten Jahrzehnt Eingang in verschiedene technische Gebiete gefunden, in denen elektrooptische und Anzeigevorrichtungs-Eigenschaften gefragt sind (z.B. in Uhren-, Taschenrechner- und Schreibmaschinenanzeigen). Diese Anzeigevorrichtungen beruhen auf den dielektrischen Ausrichtungseffekten in den nematischen, cholesterischen und smektischen Phasen der flüssigkristallinen Verbindungen, wobei - verursacht durch die dielektrische Anisotropie - die molekulare Längsachse der Verbindungen eine bevorzugte Ausrichtung in einem angelegten elektrischen Feld einnimmt. Die üblichen Schaltzeiten bei diesen Anzeigevorrichtungen sind für viele andere potentielle Anwendungsgebiete von Flüssigkristallen, die an sich für die Technik wegen ihrer einzigartigen Eigenschaften sehr vielversprechende chemische Verbindungen sind, zu lang. Dieser Nachteil macht sich insbesondere dann bemerkbar, wenn eine große Anzahl von Bildpunkten angesteuert werden muß, wodurch die Herstellungskosten von Geräten, die größere Flächen enthalten, z.B. Videogeräte, Oszillographen oder Fernseh-, Radar-, EDV- oder Schreibautomaten-Bildschirme, zu hoch werden.

Neben den nematischen und cholesterischen Flüssigkristallen haben seit einigen wenigen Jahren in zunehmendem Maß auch optisch aktive, smektische Flüssigkristall-Phasen an Bedeutung gewonnen.

Clark und Lagerwall konnten zeigen, daß die Verwendung ferroelektrischer Flüssigkristallsysteme in sehr dünnen Zellen zu optoelektrischen Schalt- oder Anzeigeelementen führt, die im Vergleich mit den herkömmlichen TN ("twisted nematic")-Zellen um bis zu einem Faktor 1000 schnellere Schaltzeiten haben (vgl. z.B. Lagerwall et al. "Ferroelectric Liquid Crystals for Displays", SID Symposium, October Meeting 1985, San Diego, Ca., USA). Aufgrund dieser und anderer günstiger Eigenschaften, z.B. der bistabilen Schaltmöglichkeit und des nahezu blickwinkelunabhängigen Kontrastes, sind ferroelektrische Flüssigkristalle grundsätzlich für die obengenannten Anwendungsgebiete, z.B. über eine Matrixansteuerung, gut geeignet. Man benötigt dazu entweder Verbindungen, die geneigt-smektische Phasen ausbilden und selbst optisch aktiv sind, oder man kann durch Dotierung von Verbindungen, die zwar solche smektischen Phasen ausbilden, selbst aber nicht optisch aktiv sind, mit optisch aktiven Verbindungen chirale, geneigt-smektische Phasen induzieren. Die gewünschte Phase soll dabei über einen möglichst großen Temperaturbereich stabil sein.

Zur Erzielung eines guten Kontrastverhältnisses in elektrooptischen Bauelementen ist eine einheitliche planare Orientierung der Flüssigkristalle nötig. Eine gute Orientierung in der $S_A$*- und $S_C$*-Phase läßt sich erreichen, wenn die Phasenfolge der Flüssigkristallmischung mit abnehmender Temperatur lautet:

Isotrop $\rightarrow$ N* $\rightarrow$ $S_A$* $\rightarrow$ $S_C$*.

Voraussetzung ist, daß der pitch (Ganghöhe der Helix) in der N*-Phase sehr groß (> 10 µm) oder noch besser völlig kompensiert ist. (T. Matsumoto et al., p. 468-470, Proc. of the 6th Int. Display Research Conf., Japan Display, Sept. 30 - Oct. 2, 1986, Tokyo, Japan; M. Murakami et al., ibid. p. 344 - p. 347). Dies erreicht man, indem man zu der chiralen Flüssigkristallmischung, die in der N*-Phase z.B. eine linksdrehende Helix aufweist, einen weiteren optisch aktiven Dotierstoff, der eine rechtsdrehende Helix induziert, in solchen Mengen hinzugibt, daß die Helix gerade oder wenigstens annähernd (Ganghöhe > 10 µm) kompensiert wird.

Aus dem Stand der Technik sind zwar schon Verbindungen bekannt, die einige der vorstehend beschriebenen Eigenschaften aufweisen, da aber die Herstellung praxisgerechter Flüssigkristallmischungen - je nach Art und Anzahl der Komponenten - ein in hohem Maße komplexer Vorgang ist, wird eine breite Auswahl an Dotierstoffen benötigt. Insbesondere werden solche Verbindungen gesucht, die bei geringer Zusatzmenge ein hohes Verdrillungsvermögen aufweisen und die vorstehend beschriebene Kompensation bewirken, ohne dabei die übrigen geforderten und deshalb "komponierten" Eigenschaften der Mischungen (z. B. eine hohe spontane Polarisation Ps) negativ zu beeinflussen.

Aus der DE-A 37 13 273 sind optisch aktive 1,3-Dioxolan-4-carbonsäureester mit den von der Flüssigkristall-Chemie her bekannten mesogenen Gruppierungen im Alkohol- bzw. Phenolteil der Verbindungen bekannt. In Kristallografiya 33 (1988) 701-706 sind 2-(1,3-Dioxolan)essigsäureester beschrieben, die einen mesogenen Alkohol/Phenolteil besitzen. In der nicht vorveröffentlichten, prioritätsälteren DE-A 38 24 902 werden 1,3-Dioxolan-Derivate, die in 4-Stellung einen mesogenen Rest tragen, vorgestellt, wobei der mesogene Rest über eine Oximethylen- oder eine Thiomethylengruppe mit dem Dioxolanring verknüpft ist.

Aufgabe der vorliegenden Erfindung ist es, neue Verbindungen mit den vorstehend beschriebenen Eigenschaften bereitzustellen, die über den Rahmen der aus dem Stand der Technik bekannten Verbindungen hinaus mit den unterschiedlichsten Flüssigkristallmischungen veträglich sind.

Die Erfindung geht von den bekannten, in 4-Stellung einen mesogenen Rest aufweisenden 1,3-Dioxolan-Derivaten aus.

Die erfindungsgemäßen Derivate unterscheiden sich strukturell von den im Stand der Technik genannten Verbindungen durch eine Ethylgruppe bzw. Ethenylgruppe zwischen Dioxolanring und mesogenen Ring. Sie werden durch die allgemeine Formel (I) beschrieben,

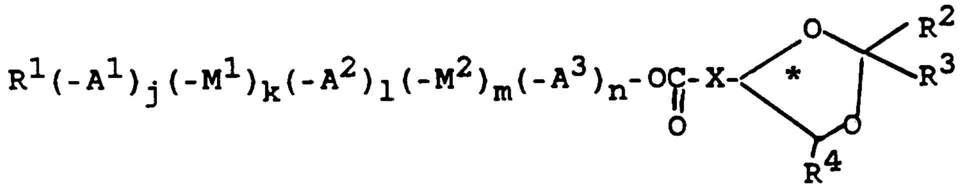

(I)

in der die Symbole folgende Bedeutung haben:

$R^1$

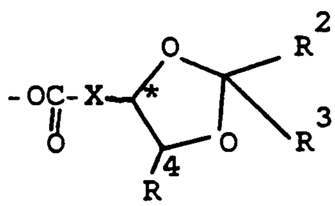

oder ein geradkettiger oder verzweigter Alkylrest mit 1 bis 16 C-Atomen oder ein geradkettiger oder verzweigter Alkenylrest mit 3 bis 16 C-Atomen, wobei diese Reste selbst asymmetrische C-Atome enthalten können, wobei eine oder mehrere nicht benachbarte $-CH_2$-Gruppen durch -O-, -S-, -CO-, -O-CO-,

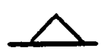 , 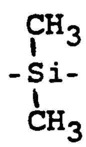

und/oder -CO-O- und wobei ein oder mehrere H durch F, Cl, Br oder CN ersetzt sein können

| | |
|---|---|
| $R^2$ und $R^3$ | jeweils H oder ein Alkylrest mit 1 bis 10 C-Atomen, wobei ein oder mehrere H des Alkylrestes durch F ersetzt sein können, oder $R^2$ und $R^3$ bilden zusammen mit dem C(2)-Atom des Dioxolanrings einen Cyclopentan-, Cyclohexan- oder Cycloheptanring oder eine Ketogruppe |
| $R^4$ | H oder ein Alkylrest mit 1 bis 10 oder ein Alkenylrest mit 2 bis 10 C-Atomen |
| j und l | null, 1 oder 2 |
| k und m | null oder 1 |
| n | null, 1 oder 2 mit folgender Maßgabe: wenn j und/oder l = null sind, ist k = null; wenn n = null ist, ist m = null; die Summe j + l + n ist minimal 1 und maximal 3, |

$-A^1$ und $-A^2$

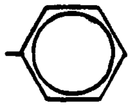 , 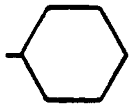 , 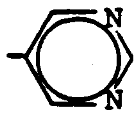 , 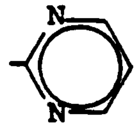 ,

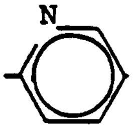 , 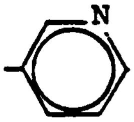 , 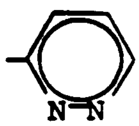 , 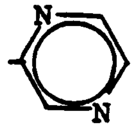 ;

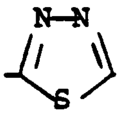 , 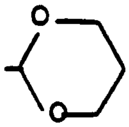 , 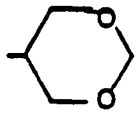 , 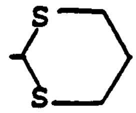 ,

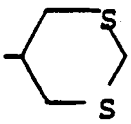 , 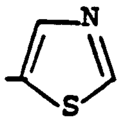 , 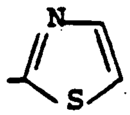 , 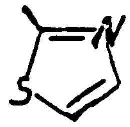 ,

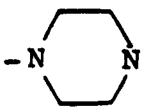

-A$^3$

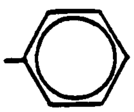 , 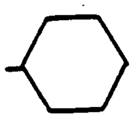 , 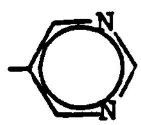 , 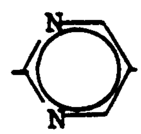 ,

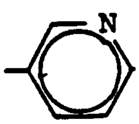 , 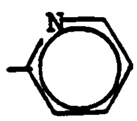 , 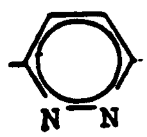

wobei jeder der Reste ($A^1$, $A^2$, $A^3$) mit einem oder mehreren F, Cl, Br oder CN substituiert sein kann -$M^1$ und -$M^2$ -CO-O, -O-CO, -$CH_2CH_2$, -CH=CH, -$CH_2O$, -$OCH_2$, -C≡C

X $CH_2$-$CH_2$, CH=CH

Die obigen Formeln sind dabei so zu verstehen, daß die nächste Gruppe am äußersten rechten Molekülteil anschließt.

In einer bevorzugten Ausfuhrungsform haben die Symbole in der allgemeinen Formel (I) die folgende Bedeutung:

| | |
|---|---|
| $R^1$ | ein geradkettiger oder verzweigter Alkyl- oder Alkenylrest mit 4 bis 14 C-Atomen, der ein asymmetrisches C-Atom enthalten kann, oder wobei eine -$CH_2$-Gruppe durch -O-, S oder -O-CO- ersetzt sein kann, oder wobei ein der mehrere H durch F ersetzt sein können, |
| $R^2$,$R^3$,$R^4$ | H oder ein Alkylrest mit 1 bis 5 C-Atomen oder $R^2$,$R^3$ zusammen mit dem C(2)-Atom des Dioxolanrings einen Cyclopentan- oder Cyclohexanring oder eine Ketogruppe |
| j und l | null oder 1, |
| k,m,n | null oder 1 |
| -$M^1$,-$M^2$ | -CO-O-, -O-CO |

X $CH_2$-$CH_2$, CH = CH

In einer weiteren bevorzugten Ausführungsform haben die 1,3-Dioxolan-Derivate die allgemeine Formel (IV)

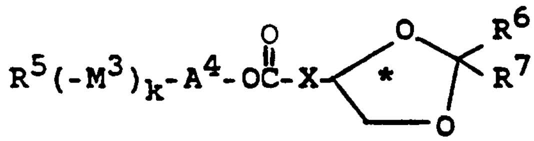

(IV)

worin bedeuten:

X $CH_2$-$CH_2$, CH = CH,

$R^6$, $R^7$ Methyl oder zusammen mit dem C(2)-Atom des Dioxolanrings einen Cyclohexanring,

$R^5$ einen geradkettigen oder verzweigten Alkyl- oder Alkenylrest mit 6 bis 12 C-Atomen, der ein asymmetrisches C-Atom enthalten kann,

$-M^3$ -O, -S oder -O-CO,

$-A^4$

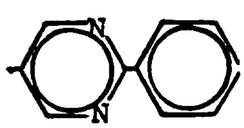 , 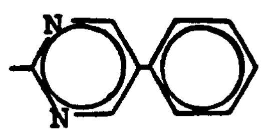 , 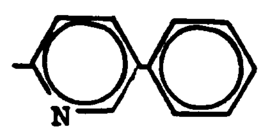 ,

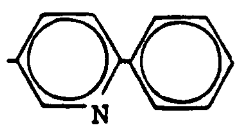 , 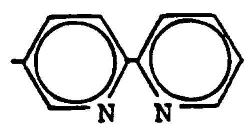 , 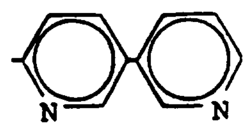 ,

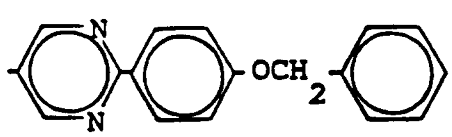 , 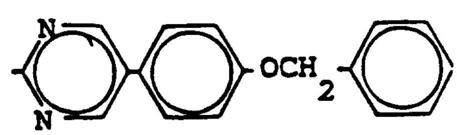 ,

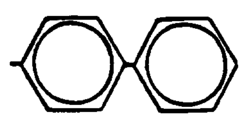 , 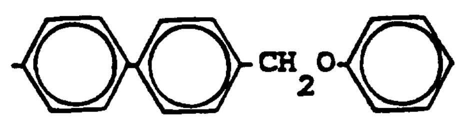 ,

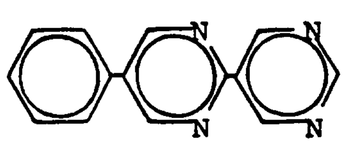 , 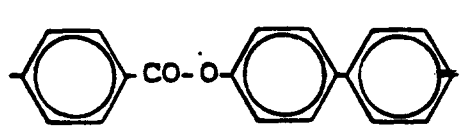

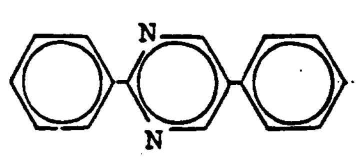 oder 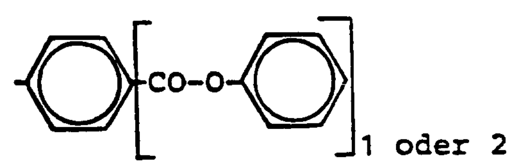

Zu den neuen Verbindungen der allgemeinen Formel (I), insbesondere (IV), gehören bevorzugt die in den Beispielen namentlich genannten Verbindungen.

Zur Herstellung der Verbindungen der allgemeinen Formel (I) werden mesogene Alkohole der allgemeinen Formel (II)

$R^1(-A^1)_j(-M^1)_k(-A^2)_l(-M^2)_m(-A^3)_n$-O-H (II)

mit Carbonsäuren des Typs III

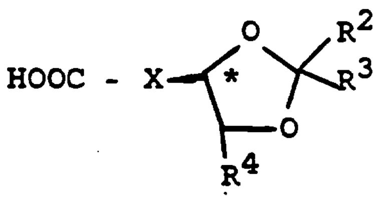

(III)

wobei X = $CH_2$-$CH_2$, CH = CH umgesetzt.

Die Veresterung von II und III erfolgt in Gegenwart von wasserbindenden Mitteln oder Kondensationsreagentien wie N,N-Carbonyldiimidazol, Dicyclohexylcarbodiimid oder Azodicarbonsäureester/Triphenylphosphin.

Methoden zur Herstellung von Verbindungen des Typs (II) sind dem Fachmann bekannt (z. B. Zaschke et al., Flüssige Kristalle in Tabellen, Bände I und II, VEB Deutscher Verlag für Grundstoffindustrie, Leipzig, 1984).

Die Carbonsäuren III können nach Literaturstellen wie Synthesis 1986, 403 hergestellt werden. Die Reinigung der Verbindungen (I) kann in der Regel durch Chromatographie und/oder Kristallisation erfolgen.

Die eine weitere Lösung der gestellten Aufgabe darstellenden, insbesondere ferroelektrischen Flüssigkristallmischungen bilden Flüssigkristall-Phasen und enthalten mindestens eine optisch aktive Verbindung der allgemeinen Formel (I).

Unter dem Begriff "Flüssigkristallphase" sind nematische, cholesterische, orthogonal smektische oder geneigt ("tilted")-smektische, insbesondere $S_c$-Phasen zu verstehen. Die Flüssigkristallmischungen bestehen aus 2 bis 20, vorzugsweise 2 bis 15 Komponenten, darunter mindestens eine der erfindungsgemäß beanspruchten chiralen Verbindungen.

Die anderen Bestandteile werden vorzugsweise ausgewählt aus den bekannten Verbindungen mit nematischen, cholesterischen und/oder smektischen Phasen; dazu gehören beispielsweise Schiffsche Basen, Biphenyle, Terphenyle, Phenylcyclohexane, Cyclohexylbiphenyle, N, S oder O-haltige Heterocyclen, z.B. Pyrimidine, Zimtsäureester, Cholesterinester oder verschieden überbrückte, terminalpolar mehrkernige Ester von p-Alkylbenzoesäuren. Im allgemeinen liegen die im Handel erhältlichen Flüssigkristallmischungen bereits vor der Zugabe der optisch aktiven Verbindung(en) als Gemische verschiedenster Komponenten vor, von denen mindestens eine mesogen ist, d.h. als Verbindung, in derivatisierter Form oder im Gemisch mit bestimmten Cokomponenten eine Flüssigkristallphase zeigt, die mindestens eine enantiotrope (Klärtemperatur > Schmelztemperatur) oder monotrope (Klärtemperatur < Schmelztemperatur) Mesophasenbildung erwarten läßt.

Insbesondere enthält die Flüssigkristall-Mischung neben mindestens einer der erfindungsgemäß beanspruchten optisch aktiven Verbindungen eine Esterverbindung mit $S_c$-Phase, z.B. einen Alkoxybenzoesäurephenylester, oder eine zweikernige aromatische Verbindung mit einem stickstoffhaltigen Heterocyclus, z.B. ein Alkylpyrimidinyl-alkoxy-benzol.

Von der oder den erfindungsgemäßen Verbindung(en) enthalten die Flüssigkristallmischungen im allgemeinen 0,05 bis 70 Gew.-%, insbesondere 0,1 bis 50 Gew.-%.

Das hohe Verdrillungsvermögen der erfindungsgemäßen Verbindungen führt bereits in nematischen Phasen bei den eher "klassischen" Displaytechnologien zu vorteilhaften Einsatzmöglichkeiten. Hierbei steht jedoch häufig nicht die Kompensation, sondern die Erzielung einer Verdrillung durch eine möglichst geringe Zugabemenge an chiralem Dotierstoff im Vordergrund. Dies gilt sowohl für die z. Z. (noch) marktbeherrschende TN ("twisted-nematic")-Technologie [M. Schadt et al., Appl. Phys. Lett 18, 127 (1971)] als auch für das sogenannte White-Taylor-Display [D. L. White et al., J. Appl. Phys. 45, 4718 (1974)] oder das SBE/STN ("super-birefringence-effect"/"super-twisted-nematic")-Display [T. J. Scheffer et al., Appl. Phys. Lett. 45, 1021 (1984)] und seine verschiedenen Modifikationen wie das OMI ("optical mode interference")Display [M. Schadt et al., Appl. Phys. Lett. 50, 236 (1987)].

Die erfindungsgemäßen Verbindungen sind als Dotierstoffe für - insbesondere ferroelektrische - Flüssigkristallmischungen mit nematischen, cholesterischen und/oder smektischen Phasen geeignet. Sie weisen

beispielsweise in nematischen bzw. cholesterischen Phasen ein hohes Verdrillungsvermögen auf, wobei bereits geringe Zugabemengen ausreichen, die eingangs beschriebene Helixkompensation durchzuführen, ohne dabei andere bereits vorhandene Eigenschaften der dotierten Mischung wesentlich negativ zu beeinflussen.

Dieses gilt in unterschiedlichem Maße für die erfindungsgemäßen Verbindungen mit X = $CH_2$-$CH_2$ und X = CH=CH in Formel (I). Weil die Verbindungen mit X = $CH_2$-$CH_2$ keine meßbare spontane Polarisation besitzen, eignen sie sich hervorragend zur Feinabstimmung der Helixganghöhe, ohne daß die spontane Polarisation der Mischung durch ihre Zugabe merklich beeinflußt wird. Die Verbindungen gemäß Formel (I) mit X= HC=CH lassen sich wegen ihres erheblichen Verdrillungsvermögens und nur mäßig hoher spontaner Polarisation bereits bei sehr geringer Zugabe ebenfalls für diesen Zweck einsetzen.

Darüber hinaus können sie bei höherer Konzentration auch vorteilhaft als Dotierstoff zur Erzielung einer spontanen Polarisation eingesetzt werden, insbesondere in Kombination mit einem zweiten Dotierstoff mit umgekehrtem Helixdrehsinn oder einer Kombination aus mehreren Dotierstoffen mit umgekehrtem Drehsinn.

Auch in der $S_c$-Phase induzieren die erfindungsgemäßen Verbindungen eine Helix, so daß dieser Effekt benutzt werden kann, eine Verdrillung in der $S_c^*$-Phase zu kompensieren oder auf einen bestimmten Wert einzustellen, was für den praktischen Einsatz vorteilhaft ist [z. B. T. Tsuchiga et al., Jpn. J. Appl. Phys. 25, L-27 (1986)].

**Beispiel 1**

(S)-3-(2,2-Cyclohexyliden-1,3-dioxolan-4-yl)-acrylsäure-[4-(5-octyl-pyrimidin-2-yl)-phenyl]-ester

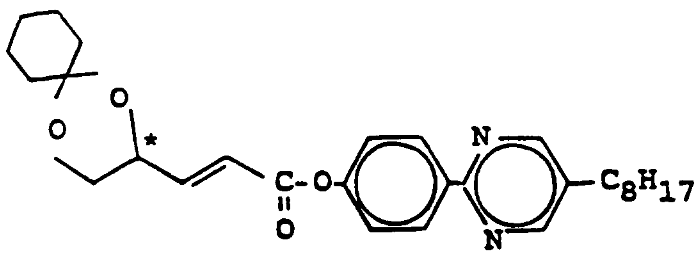

Zur Lösung von 0,76 g Dicyclohexylcarbodiimid und 0,1 g 4-Dimethylaminopyridin in 40 ml Dichlormethan werden bei RT 1,02 g 4-(5-Octyl-pyrimidin-2-yl)-phenol und 0,78 g 4,5-O-Cyclohexyliden-S-4,5-dihydroxy-2-pentensäure gegeben. Die Lösung wird 18 h bei 25 °C gehalten, der Niederschlag abgesaugt und das Filtrat vom Lösungsmittel befreit. Der Rückstand wird chromatographisch aufgetrennt ($SiO_2$, Hexan : Essigester = 9 : 1). Nach Umkristallisation aus Hexan werden 0,6 g Produkt erhalten.
Schmelzpunkt: 58,4 °C,

$$[\alpha]^{22}_{365} = +138,9^\circ \quad (c = 2,\ CH_2Cl_2).$$

**Beispiel 2**

(S)-3-(2,2-Cyclohexyliden-1,3-dioxolan-4-yl)-acrylsäure-[5-(2-(4-octyloxyphenyl))-pyrimidin]-ester

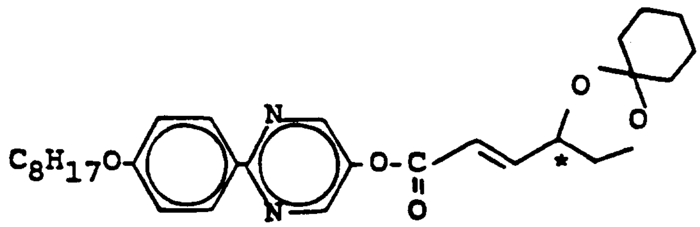

Schmelzpunkt: 97,7°C, $[\alpha]^{22}_{365}$ = +164,32° (c = 2, $CH_2Cl_2$).

**Beispiel 3**

(S)-3-(2,2-Cyclohexyliden-1,3-dioxolan-4-yl)-acrylsäure-[4-(5-octyloxy-pyrimidin-2-yl)-phenyl]-ester

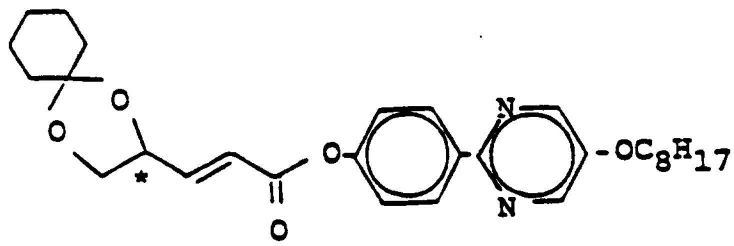

K [50 $S_A$ 56,5 N* 64] 71,1 I, $[\alpha]^{22}_{365}$ = +139,31°, (c = 2, $CH_2Cl_2$)

**Beispiel 4**

(S)-3-(2,2-Cyclohexyliden-1,3-dioxolan-4-yl)-acrylsäure-[4-(2-octyloxy-pyrimidin-5-yl)-phenyl]-ester

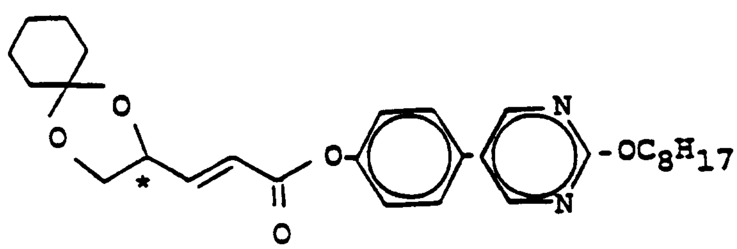

K [63 $S_A$ 75,3] 85,9 I, $[\alpha]^{22}_{365}$ = +127,88°, (c = 2, $CH_2Cl_2$).

**Beispiel 5**

(S)-3-(2,2-Cyclohexyliden-1,3-dioxolan-4-yl)-acrylsäure-[4-(4-Octyloxy-benzoyloxy)-phenyl]-ester

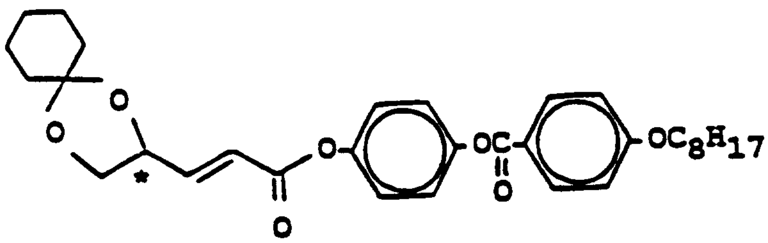

K [79 N*] 93,8 I, $[\alpha]^{22}_{365}$ = +101,96° (c = 2, $CH_2Cl_2$).

Analog, jedoch unter Verwendung von 4,5-O-Cyclohexyliden-S-4,5-dihydroxy-pentansäure worden erhalten:

**Beispiel 6**

(S)-3-(2,2-Cyclohexyliden-1,3-dioxolan-4-yl)-propionsäure-[4-(5-octyl-pyrimidin-2-yl)-phenyl]-ester

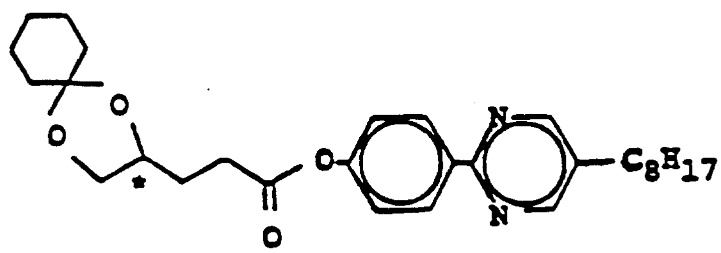

Schmelzpunkt: 48,2°C, $[\alpha]^{23}_{365}$ = -14,25° (c = 2, $CH_2Cl_2$).

**Beispiel 7**

(S)-3-(2,2-Cyclohexyliden-1,3-dioxolan-4-yl)-propionsäure-[4-(5-octyloxy-pyrimidin-2-yl)-phenyl]-ester

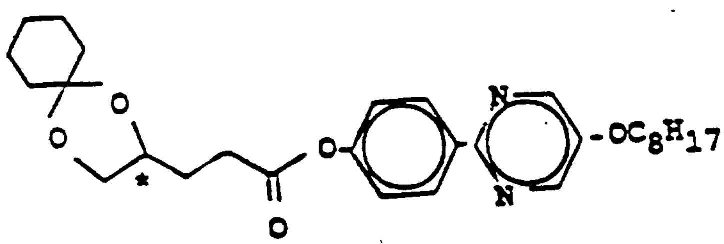

K [$S_A$ 60°] 72,4 I, $[\alpha]^{22}_{365}$ = -13,88° (c = 2, $CH_2Cl_2$).

**Beispiel 8**

(S)-3-(2,2-Cyclohexyliden-1,3-dioxolan-4-yl)-propionsäure-[5-(2-(4-octyloxyphenyl))-pyrimidin]-ester

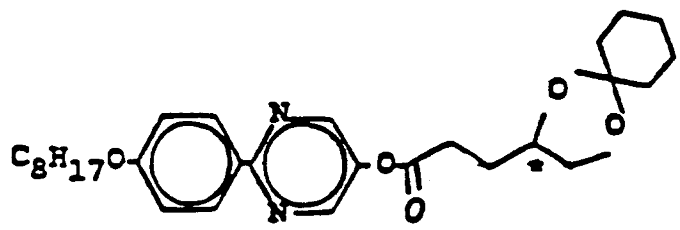

K [$S_A$ 48°] 90,1 I, $[\alpha]^{22}_{365}$ = -17,81° (c = 2, $CH_2Cl_2$).

**Beispiel 9**

(S)-3-(2,2-Cyclohexyliden-1,3-dioxolan-4-yl)-propionsäure-[4-(2-octyloxy-pyrimidin-5-yl)-phenyl]-ester

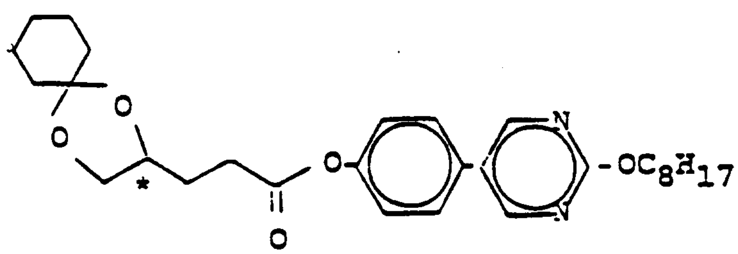

Schmelzpunkt: $[\alpha]^{22}_{365}$ = -12,70, (c = 2, $CH_2Cl_2$).

Meßmethode:
Versetzt man ein (nicht-chirales) Lösemittel mit einer kleinen Menge einer chiralen Verbindung, so wird die Ebene des linear polarisierten Lichts um den (charakteristischen) Winkel $\alpha$ gedreht; dieser Winkel wird wie folgt angegeben: $[\alpha]^T$ , (c = x, LM), wobei die Symbole folgende Bedeutung haben:
x = Konzentration der Lösung in g/l, LM = Lösemittel,
T = Temperatur der Lösung. Der Drehwinkel wird in einem Polarimeter nach 10 cm Durchgang des Lichts bestimmt.

**Anwendungsbeispiele A1 bis A7**

Die Bestimmung der HTP ("helical twisting power")-Werte, d. h. einem Maß für das Verdrillungsvermögen, wird nach dem bei Kassubek et al., Mol. Cryst. Liq. Cryst. 8, 305-314 (1969) beschriebenen Verfahren durchgeführt. Dazu werden die chiralen Dotierstoffe in eine nematische Wirtsmischung eingemischt; mit dieser Testmischung wird eine planar-orientierende Keilzelle gefüllt und durch Ausmessen der Versetzungslinien im Polarisationsmikroskop (bei bekanntem Keilwinkel der Zelle) die cholesterische Helixganghöhe bestimmt. Das Vorzeichen der Ganghöhe kann durch Drehen des Analysators bestimmt werden. Ist die Ganghöhe (oder "pitch") p bekannt, so berechnet man die HTP nach: HTP = 1/p•X mit X = Molenbruch des chiralen Dotierstoffes. Die HTP-Werte sind der Tabelle 1 zu entnehmen. Die Wirtsmischung besitzt folgenden Phasenbereich:
X 10 $Sm_C$ 84 $Sm_A$ 93 N 105 I
Durch Zugabe der Beispielsubstanzen wird der nematische Phasenbereich beeinflußt, so daß Messungen zwischen 80 ° C und 102 ° C möglich werden.

Die Ergebnisse zeigen, daß große HTP-Werte erhältlich sind und damit ein weiter Bereich an gewünschten Ganghöhen durch geringe Zugabemengen der erfindungsgemäßen Verbindungen ermöglicht wird.

Für die gebrauchsfertigen ferroelektrischen Flüssigkristall-Mischungen wurden die Werte für die spontane Polarisation $P_s$ [nC/cm$^2$], und die optische Schaltzeit $\tau$ [$\mu$s], ermittelt (alle Messungen bei 25 °C).

Die $P_s$-Werte werden nach der Methode von H. Diamant et al. (Rev. Sci. Instr., 28, 30, 1957) gemessen, wobei Meßzellen mit 2 $\mu$m Elektrodenabstand und geriebenem Polyimid als Orientierungsschicht verwendet werden.

Zur Bestimmung von $\tau$ wird die Meßzelle auf dem Drehtisch eines Polarisationsmikroskops zwischen gekreuztem Analysator und Polarisator befestigt.

Mit Hilfe einer Photodiode erfolgt die Bestimmung der Schaltzeit $\tau$, indem die Anstiegzeit des Lichtsignals von 10 auf 90 % Signalhöhe gemessen wird. Die Schaltspannung besteht aus Rechteckpulsen und beträgt $\pm$ 10 V/$\mu$m.

Die Phasenumwandlungstemperaturen werden beim Aufheizen mit Hilfe eines Polarisationsmikroskops anhand der Texturänderungen bestimmt. Die Bestimmung des Schmelzpunkts wurde hingegen mit einem DSC-Gerät durchgeführt. Die Angabe der Phasenumwandlungstemperaturen zwischen den Phasen
Nematisch (N bzw. N*)
Smektisch-C ($S_c$ bzw. $S_c$*)
Smektisch-A ($S_A$ bzw. $S_A$*)
Kristalling (X)
erfolgt in °C und die Werte stehen zwischen den Phasenbezeichnungen in der Phasenfolge.

**Anwendungsbeispiel A8**

Eine erfindungsgemäße Mischung besteht aus den folgenden acht Komponenten

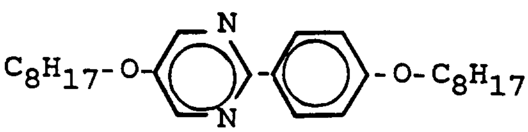

11,11 Mol-%

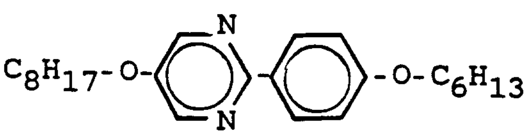

24,06 Mol-%

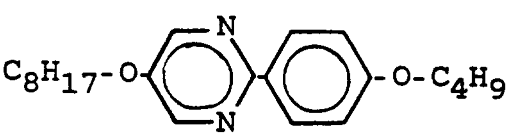

25,38 Mol-%

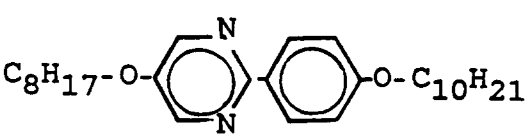

20,18 Mol-%

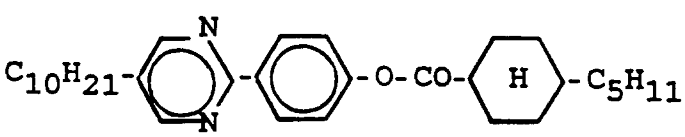

14,25 Mol-%

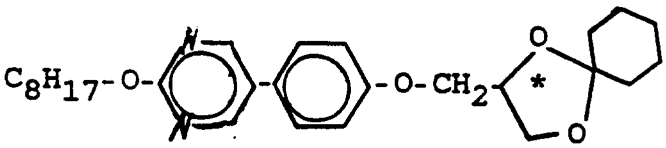

0,5 Mol-%

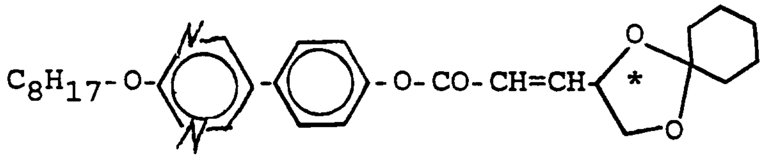

0,3 Mol-%

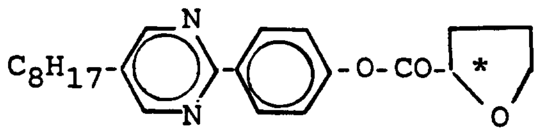

4,2 Mol-%

und weist folgende Phasenfolge auf

$S_c$* 78,4 $S_A$* 88,4 N 101 I

Die Polarisation der Mischung bei einer Temperatur von 25 °C beträgt 7,8 nC $cm^{-2}$ und die Schaltzeit $\tau$ beträgt bei 25 °C 112 $\mu$s.

Der Pitch dieser Mischung ist über den gesamten nematischen Phasenbereich größer als 50 $\mu$m.

Dieses Beispiel belegt, daß die erfindungsgemäße Verbindung in besonderer Weise für ferroelektrische Mischungen geeignet sind, bei denen der Pitch über einen breiten Phasenbereich kompensiert sind.

**Anwendungsbeispiel A9**

Eine erfindungsgemäße ferroelektrische Mischung besteht aus den folgenden 11 Verbindungen:

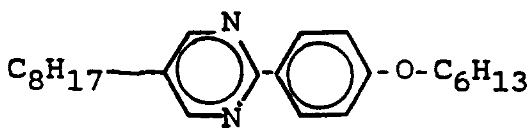

14,54 Mol-%

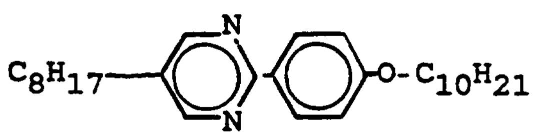

9,70 Mol-%

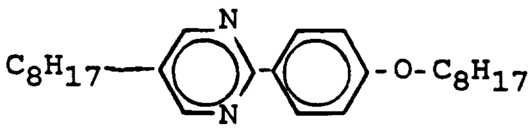 13,05 Mol-%

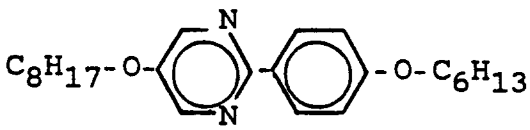 9,09 Mol-%

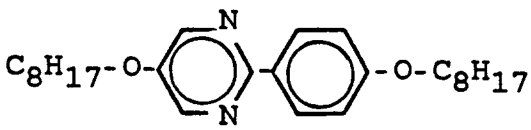 4,2 Mol-%

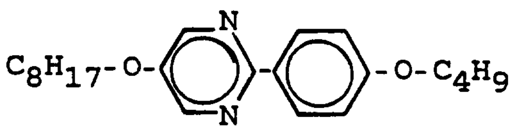 9,59 Mol-%

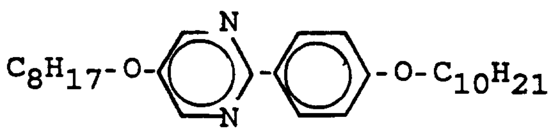 7,62 Mol-%

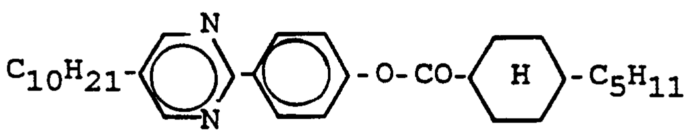 22,61 Mol-%

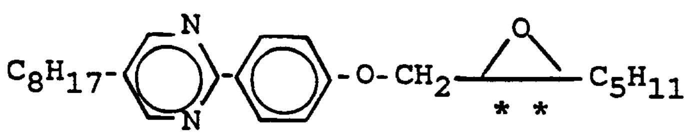 6,20 Mol-%

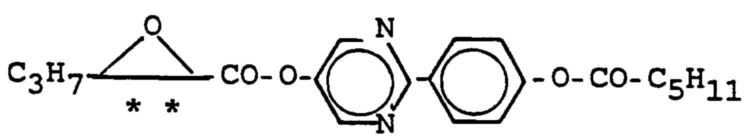 2,30 Mol-%

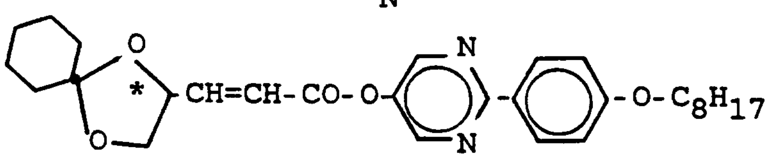 1,07 Mol-%

Sie weist folgende Phasenfolge auf
X -2 $S_c^*$ 64,5 $N^*$ 94 I
Die Mischung weist bei einer Temperatur von 25 °C eine spontane Polarisation von 21 nC $cm^{-2}$ auf und schaltet bei einer Feldstärke von 10 $V\mu m^{-1}$ mit der Schaltzeit $\tau$ = 45 $\mu s$. Diese Mischung ist über den gesamten Temperaturbereich so gut Pitch-kompensiert, daß der Betrag des Pitches größer als 50 $\mu m$ ist.

Dieses Beispiel belegt, daß die erfindungsgemäßen Komponenten für ferroelektrische Mischungen mit guter Pitchkompensation geeignet sind.

**Anwendungsbeispiel A10**

Eine erfindungsgemäße ferroelektrische Mischung besteht aus folgenden 11 Komponenten

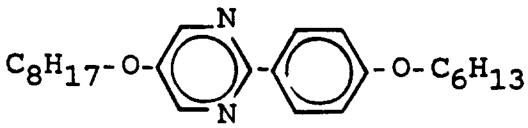

8,9 Mol-%

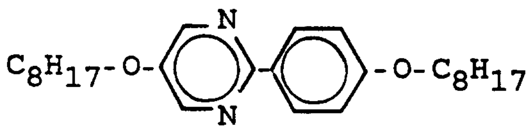

4,12 Mol-%

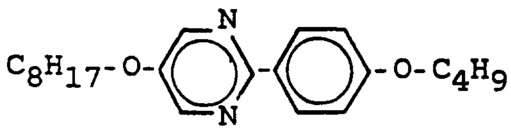

9,39 Mol-%

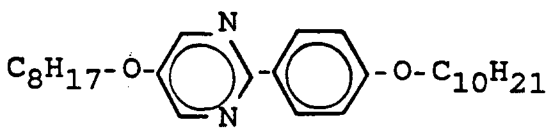

7,46 Mol-%

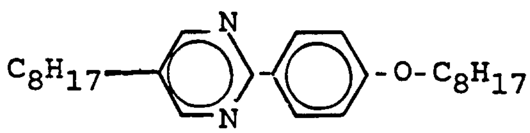

12,78 Mol-%

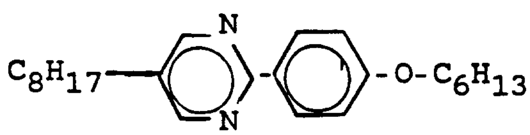

14,24 Mol-%

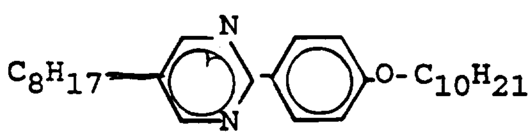

9,5 Mol-%

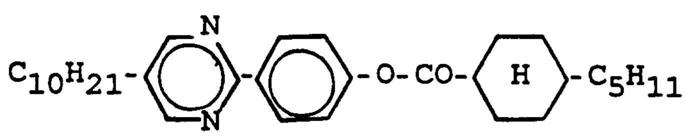

22,14 Mol-%

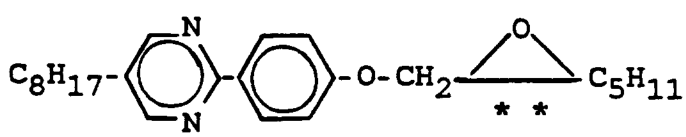

3,92 Mol-%

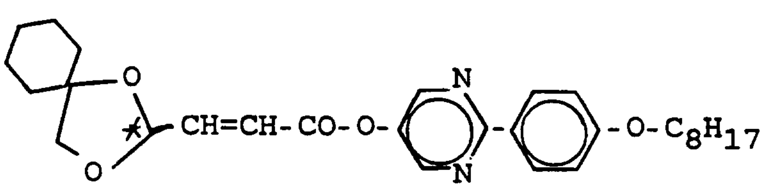

1,1 Mol-%

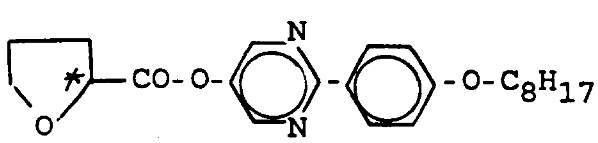

6,44 Mol-%

Sie weist folgende Phasenfolge auf

X -3 $S_c$* 61,5 N* 94 I

Die Polarisation der Mischung weist bei einer Temperatur von 25 °C beträgt 12 nC $cm^{-2}$. Diese Mischung ist über den gesamten Temperaturbereich so gut kompensiert, daß der Betrag des Pitches größer als 50 µm ist.

Dieses Beispiel belegt die besondere Eignung der erfindungsgemäßen Komponenten für ferroelektrische Mischungen mit guter Pitchkompensation.

**Tabelle 1**

| Anwendungs-beispiele | Dotierstoff aus Beispiel | HTP ($\mu m^{-1}$) (cholersterische Phase) bei 85°C | 90°C | 95°C | 100°C | Sp. Polarisation ($nC/cm^2$) (smektische $c^*$-Phase) bei 25°C |
|---|---|---|---|---|---|---|
| A1 | 1 | -- | -5,0 | -6,9 | -8,6 | +6,2 |
| A2 | 2 | -6,0 | -7,7 | -9,4 | -12 | +9,4 |
| A3 | 3 | -- | -- | -5,8 | -7,9 | +3,1 |
| A4 | 4 | -- | -- | -6,9 | -8,5 | +5,1 |
| A5 | 6 | -1,4 | -2,0 | -2,3 | -- | < 0,5 |
| A6 | 7 | -- | -1,4 | -1,9 | -- | < 0,5 |
| A7 | 8 | -2,2 | -2,7 | -3,3 | -- | < 0,5 |

## Patentansprüche

1. Optisch aktive, in 4-Stellung einen mesogenen Rest tragende 1,3-Dioxolan-Derivate der allgemeinen Formel (I)

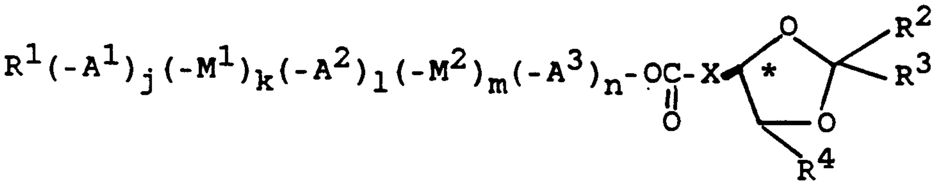 (I)

in der die Symbole folgende Bedeutung haben:

$R^1$

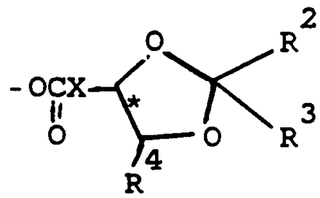

oder ein geradkettiger oder verzweigter Alkylrest mit 1 bis 16 C-Atomen oder ein geradkettiger oder verzweigter Alkenylrest mit 3 bis 16 C-Atomen, wobei diese Reste selbst asymmetrische C-Atome enthalten können, wobei eine oder mehrere nicht benachbarte $-CH_2$-Gruppen durch -O-, -S-, -CO-, -O-CO-,

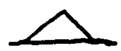,

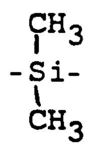

und/oder -CO-O- und wobei ein oder mehrere H durch F, Cl, Br oder CN ersetzt sein können

$R^2$ und $R^3$ jeweils H oder ein Alkylrest mit 1 bis 10 C-Atomen, wobei ein oder mehrere H des Alkylrestes durch F ersetzt sein können, oder $R^2$ und $R^3$ bilden zusammen mit dem C(2)-Atom des Dioxolanrings einen Cyclopentan-, Cyclohexan- oder Cycloheptanring oder eine Ketogruppe

$R^4$ H oder ein Alkylrest mit 1 bis 10 oder ein Alkenylrest mit 2 bis 10 C-Atomen

j und l null, 1 oder 2

k und m null oder 1

n null, 1 oder 2 mit folgender Maßgabe: wenn j und/oder l = null sind, ist k = null; wenn n = null ist, ist m = null; die Summe j + l + n ist minimal 1 und maximal 3,

$-A^1$ und $-A^2$

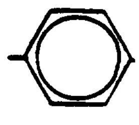 , 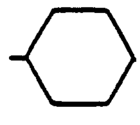 , 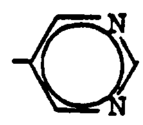 , 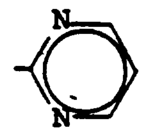 ,

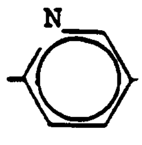 , 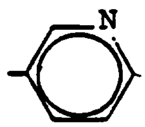 , 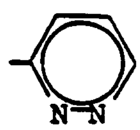 , 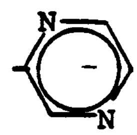 ,

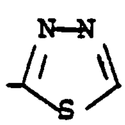 , 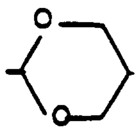 , 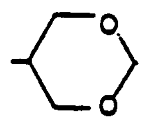 , 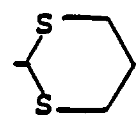 ,

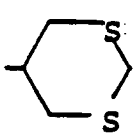 , 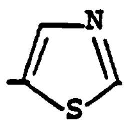 , 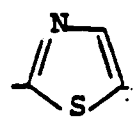 , 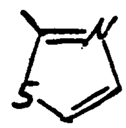 ,

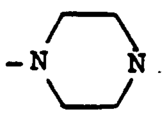

$-A^3$

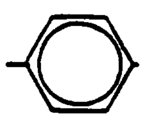 , 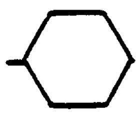 , 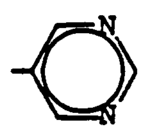 , 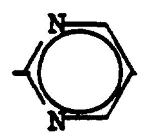 ,

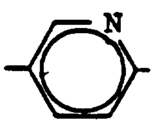 , 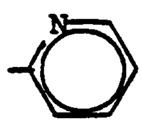 , 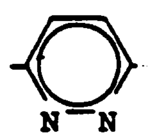

wobei jeder der Reste ($A^1$, $A^2$, $A^3$) mit einem oder mehreren F, Cl, Br oder CN substituiert sein kann

| | |
|---|---|
| $-M^1$ und $-M^2$ | -CO-O, -O-CO, $-CH_2CH_2$ , -CH = CH, $-CH_2O$, $-OCH_2$, -C≡C |
| X | CH = CH, $CH_2-CH_2$ |

**2.** 1,3-Dioxolan-Derivate nach Anspruch 1, dadurch gekennzeichnet, daß sie der allgemeinen Formel (IV) entsprechen

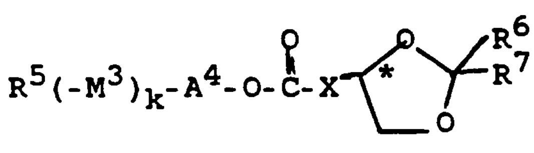

(IV)

worin bedeuten:

$R^6$, $R^7$ Methyl oder zusammen mit dem C(2)-Atom des Dioxolanrings einen Cyclohexanring

$R^5$ einen geradkettiger oder verzweigter Alkyl- oder Alkenylrest mit 6 bis 12 C-Atomen, der ein asymmetrisches C-Atom enthalten kann,

$-M^3$ -O, -S oder -O-CO,

$-A^4$

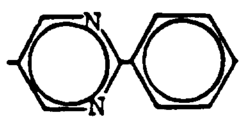 , 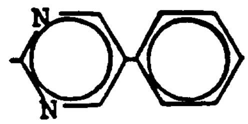 , 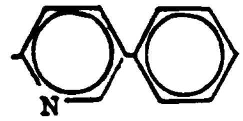 ,

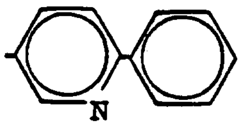 , 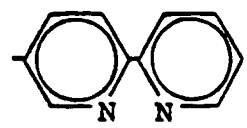 , 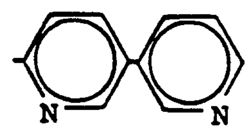 ,

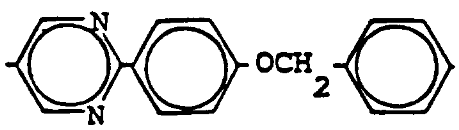 , 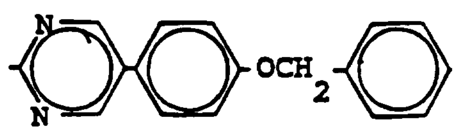 ,

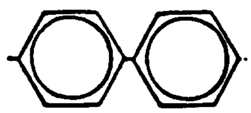 , 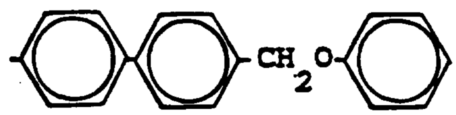 ,

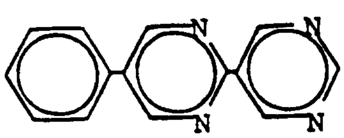 , 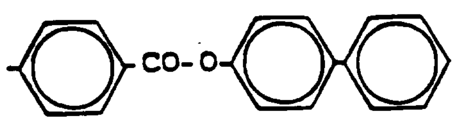

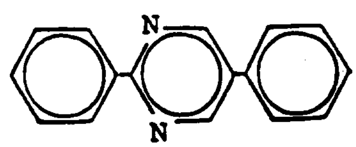 oder 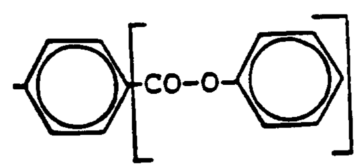 1 oder 2

3. Flüssigkristallmischung, gekennzeichnet durch einen Gehalt an mindestens einem optisch aktiven 1,3-Dioxolan-Derivat der allgemeinen Formel (I) nach Anspruch 1.

4. Ferroelektrische Flüssigkristallmischung, gekennzeichnet durch einen Gehalt an mindestens einem optisch aktiven 1,3-Dioxolan-Derivat der allgemeinen Formel (I) nach Anspruch 1.

5. Flüssigkristallmischung, gekennzeichnet durch einen Gehalt an mindestens einem optisch aktiven und mesogenen 1,3-Dioxolan-4-Derivat der allgemeinen Formel (IV) nach Anspruch 2.

6. Elektrooptisches Schalt- oder Anzeigeelement enthaltend eine Flüssigkristallmischung nach einem der Ansprüche 3 bis 5.

7. Verfahren zur Herstellung eines optisch aktiven 1,3-Dioxolan-Derivates der allgemeinen Formel (I) nach Anspruch 1, dadurch gekennzeichnet, daß mesogene Alkohole der allgemeinen (II)

$R^1(-A^1)_j(-M^1)_k(-A^2)_l(-M^2)_m(-A^3)_n$-O-H (II)

mit Dioxolansäuren (III)

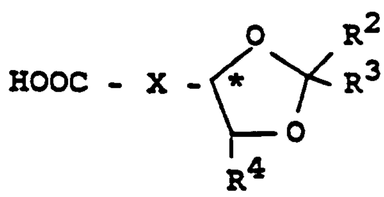 (III)

wobei X = $CH_2$-$CH_2$, CH=CH verestert werden.

## Claims

1. An optically active 1,3-dioxolane derivative, carrying a mesogenic radical in the 4-position, of the general formula (I)

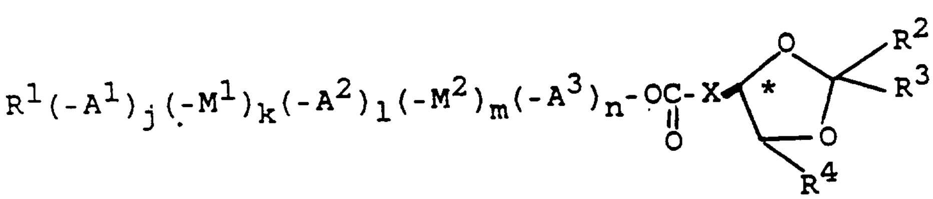 (I)

in which the symbols have the following meaning:

$R^1$ is

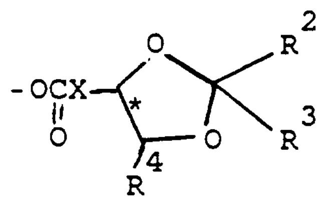

or

a straight-chain or branched alkyl radical having 1 to 16 carbon atoms or a straight-chain or branched alkenyl radical having 3 to 16 carbon atoms, it being possible for these radicals themselves to contain asymmetric carbon atoms, and it being possible for one or more non-adjacent -$CH_2$- groups to be replaced by -O-, -S-, -CO-, -O-CO-,

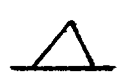, 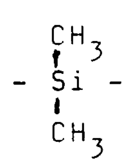

and/or -CO-O-, and it being possible for one or more H atoms to be replaced by F, Cl, Br or CN,

$R^2$ and $R^3$ are each H or an alkyl radical having 1 to 10 carbon atoms, it being possible for one or more H atoms of the alkyl radical to be replaced by F, or $R^2$ and $R^3$, together with the C(2) atom of the dioxolane ring, form a cyclopentane, cyclohexane or cycloheptane ring or a keto group,

$R^4$ is H or an alkyl radical having 1 to 10 carbon atoms or an alkenyl radical having 2 to

| | |
|---|---|
| | 10 carbon atoms, |
| j and l | are zero, 1 or 2, |
| k and m | are zero or 1, |
| n | is zero, 1 or 2, with the following proviso: k = zero if j and/or l = zero; m = zero if n = zero; the sum j + l + n is a minimum of 1 and a maximum of 3, |
| $-A^1$ and $-A^2$ | are |

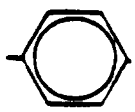 , 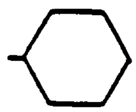 , 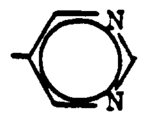 , 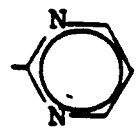 ,

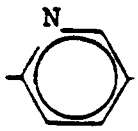 , 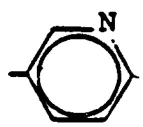 , 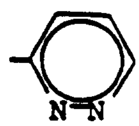 , 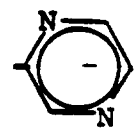 ,

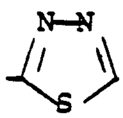 , 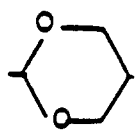 , 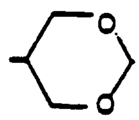 , 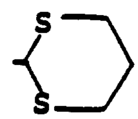 ,

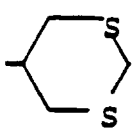 , 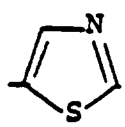 , 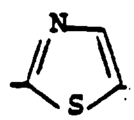 , 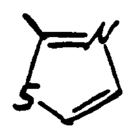 ,

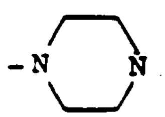

| | |
|---|---|
| $-A^3$ | is |

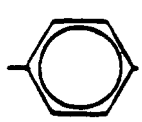 , 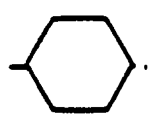 , 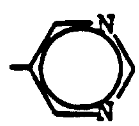 , 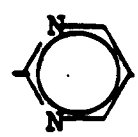 ,

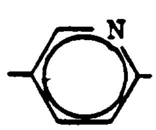 , 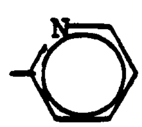 , 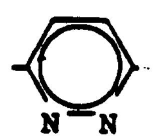

| | |
|---|---|
| | it being possible for each of the radicals ($A^1$, $A^2$, $A^3$) to be substituted by one or more F, Cl or Br atoms or CN groups, |
| $-M^1$ and $-M^2$ | are -CO-O, -O-CO, $-CH_2CH_2$, -CH=CH, $-CH_2O$, $-OCH_2$, -C≡C, and |
| X | is CH=CH or $CH_2$-$CH_2$. |

2. A 1,3-dioxolane derivative as claimed in claim 1, which corresponds to the general formula (IV)

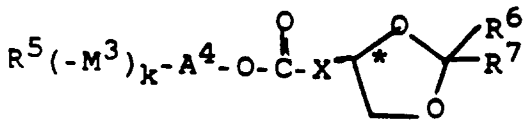 (IV)

in which:

| | |
|---|---|
| $R^6$ and $R^7$ | are methyl or, together with the C(2) atom of the dioxolane ring, are a cyclohexane ring, |
| $R^5$ | is a straight-chain or branched alkyl or alkenyl radical having 6 to 12 carbon atoms which may contain an asymmetric carbon atom, |
| $-M^3$ | is -O, -S or -O-CO, and |
| $-A^4$ | is |

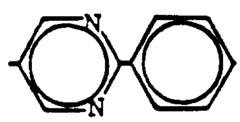 , 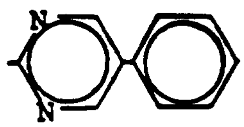 , 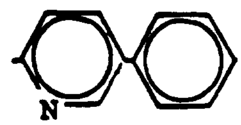 ,

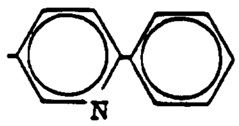 , 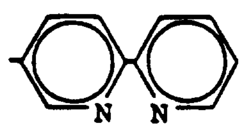 , 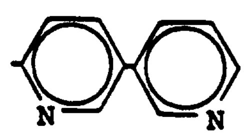 ,

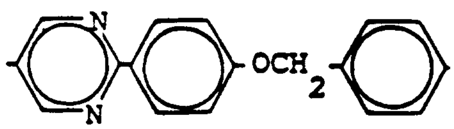 , 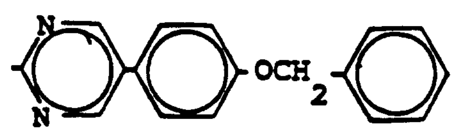 ,

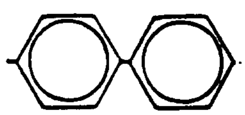 , 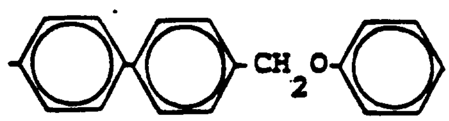 ,

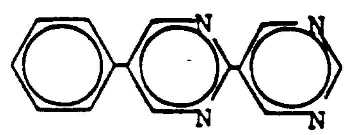 , 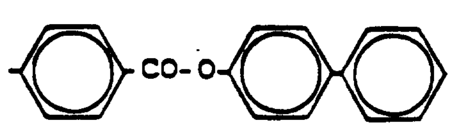 ,

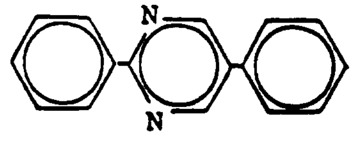 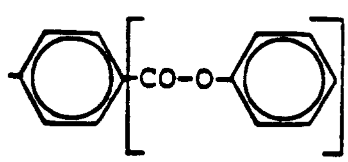

3. A liquid-crystal mixture which contains at least one optically active 1,3-dioxolane derivative of the general formula (I) as claimed in claim 1.

**4.** A ferroelectric liquid-crystal mixture which contains at least one optically active 1,3-dioxolane derivative of the general formula (I) as claimed in claim 1.

**5.** A liquid-crystal mixture which contains at least one optically active and mesogenic 1,3-dioxolane-4 derivative of the general formula (IV) as claimed in claim 2.

**6.** An electrooptical switching or display element containing a liquid-crystal mixture as claimed in any one of claims 3 to 5.

**7.** A process for the preparation of an optically active 1,3-dioxolane derivative of the general formula (I) as claimed in claim 1, which comprises esterifying a mesogenic alcohol of the general formula (II)

$R^1(-A^1)_j(-M^1)_k(-A^2)_l(-M^2)_m(-A^3)_n$-O-H (II)

using a dioxolanoic acid (III)

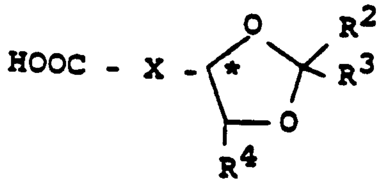

(III)

where X is $CH_2$-$CH_2$ or CH = CH.

## Revendications

**1.** Dérivés de 1,3-dioxolane optiquement actifs, portant un radical mésogène en position 4, de formule générale (I)

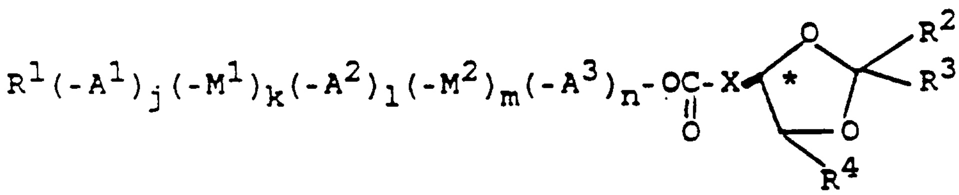

(I)

dans laquelle les symboles ont la signification suivante :

$R^1$

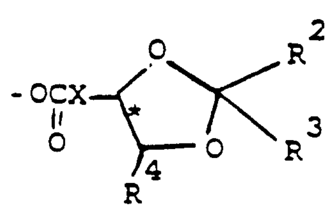

ou un radical alkyle linéaire ou ramifié comportant de 1 à 16 atomes de carbone ou un radical alcényle linéaire ou ramifié comportant de 3 à 16 atomes de carbone, ces radicaux pouvant eux-mêmes contenir des atomes de carbone asymétrique, un ou plusieurs groupes $-CH_2-$ non voisins pouvant être remplacés par -O-, -S-, -CO-, -O-CO-,

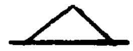 , 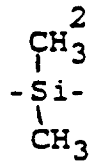

et/ou -CO-O- et un ou plusieurs H pouvant être remplacés par F, Cl, Br ou CN

$R^2$ et $R^3$ représentent chaque fois H ou un radical alkyle comportant de 1 à 10 atomes de carbone, un ou plusieurs H du radical alkyle pouvant être remplacés par F, ou $R^2$ et $R^3$ ensemble avec l'atome C(2) du cycle de dioxolane forment un cycle de cyclopentane, de cyclohexane ou de cycloheptane ou un groupe céto,

$R^4$ représente H ou un radical alkyle comportant de 1 à 10 atomes de carbone ou un radical alcényle comportant de 2 à 10 atomes de carbone,

j et l représentent 0, 1 ou 2,

k et m représentent 0 ou 1,

n représente 0, 1 ou 2

à la condition suivante :

si j et/ou l = 0, k = 0 ; lorsque n = 0, m = 0 ; la somme j+l+n est au minimum 1 et au maximum 3,

$-A^1$ et $-A^2$

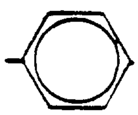 , 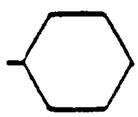 , 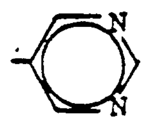 , 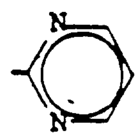 ,

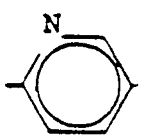 , 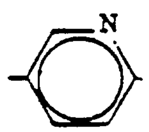 , 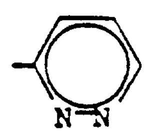 , 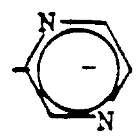 ,

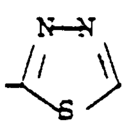 , 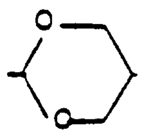 , 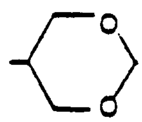 , 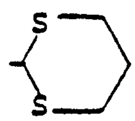 ,

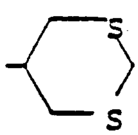 , 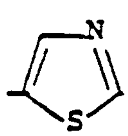 , 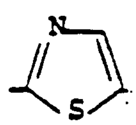 , 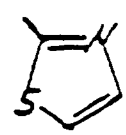 ,

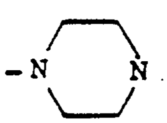

$-A^3$

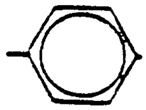 , 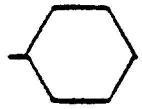 , 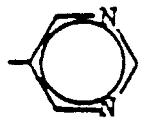 , 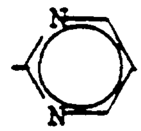 ,

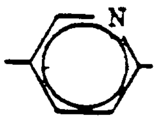 , 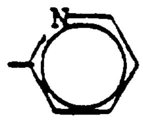 , 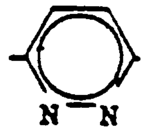

chacun des radicaux ($A^1$, $A^2$, $A^3$) pouvant être substitué par un ou plusieurs F, Cl, Br ou CN

$-M^1$ et $-M^2$ -CO-O, -O-CO, $-CH_2CH_2$, -CH = CH, $-CH_2O$, $-OCH_2$, -C≡C

X représente CH = CH, $CH_2$-$CH_2$,

**2.** Dérivés de 1,3-dioxolane selon la revendication 1, caractérisés en ce qu'ils répondent à la formule générale (IV)

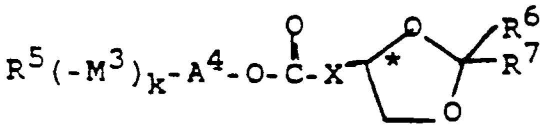 (IV)

dans laquelle :

$R^6$, $R^7$ représentent le méthyle ou, ensemble avec l'atome C(2) du cycle de dioxolane, un cycle de cyclohexane,

$R^5$ représente un radical alcényle ou alkyle linéaire ou ramifié comportant de 6 à 12 atomes de carbone, qui peuvent contenir un atome de carbone asymétrique,

$-M^3$ représente -O, -S ou -O-CO,

$-A^4$

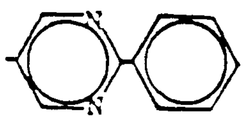 , 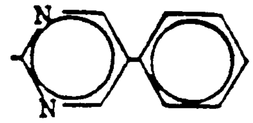 , 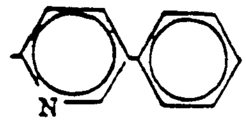 ,

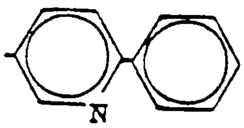 , 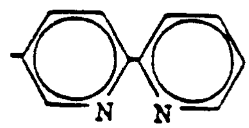 , 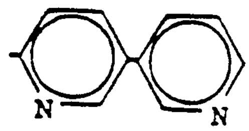 ,

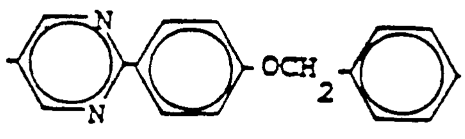 , 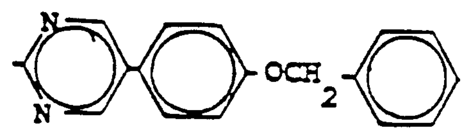 ,

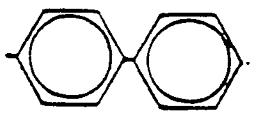 , 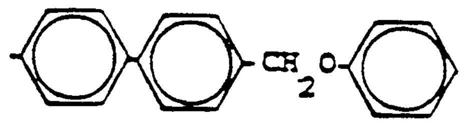 ,

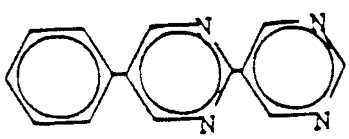 , 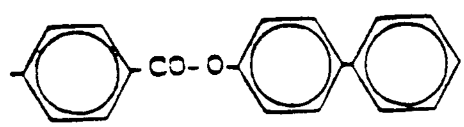

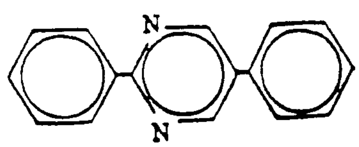 ou 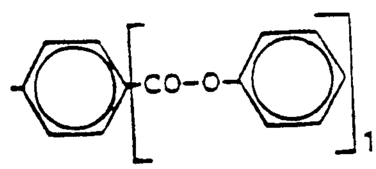 ou 2

3. Mélange de cristaux liquides caractérisé par une teneur en au moins un dérivé optiquement actif de 1,3-dioxolane de formule générale (I) selon la revendication 1.

4. Mélange de cristaux liquides ferroélectrique caractérisé par une teneur en au moins un dérivé optiquement actif de 1,3-dioxolane de formule générale (I) selon la revendication 1.

5. Mélange de cristaux liquides caractérisé par une teneur en au moins un dérivé-4 optiquement actif et mésogène de 1,3-dioxolane de formule générale (IV) selon la revendication 2.

6. Composants électro-optiques de commutation et d'affichage contenant un mélange de cristaux liquides selon l'une des revendications 3 à 5.

7. Procédé pour la préparation d'un dérivé optiquement actif de 1,3-dioxolane de formule générale (I) selon la revendication 1, caractérisé en ce qu'on estérifie des alcools mésogènes de formule générale (II)

$R^1(-A^1)_j(-M^1)_k(-A^2)_l(-M^2)_m(-A^3)_n$-O-H

avec des acides dioxolanes (III)

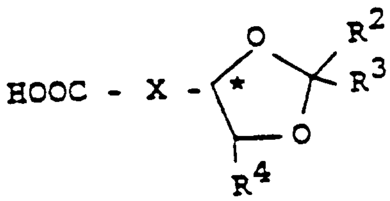

(III)

où
X = $CH_2$-$CH_2$, CH=CH.